# EUROPÄISCHE PATENTSCHRIFT

(11) **EP 3 810 036 B1**
(45) Veröffentlichungstag und Bekanntmachung des Hinweises auf die Patenterteilung: **27.11.2024**
(21) Anmeldenummer: 19735642.1
(22) Anmeldetag: 06.06.2019
(51) Int. Cl.: A61F 2/16

(54) **HALTEVORRICHTUNG ZUM HALTEN EINES OPTIKIMPLANTATS AN EINEM WANDBEREICH IN EINEM AUGENINNENRAUM EINES AUGES UND OPTIKVORRICHTUNG MIT EINER HALTEVORRICHTUNG UND EINEM OPTIKIMPLANTAT**
HOLDING APPARATUS FOR HOLDING AN OPTICAL IMPLANT AT A WALL REGION IN AN EYE INTERIOR OF AN EYE, AND OPTICAL APPARATUS COMPRISING A HOLDING APPARATUS AND AN OPTICAL IMPLANT
DISPOSITIF DE RETENUE SERVANT À MAINTENIR UN IMPLANT OPTIQUE SUR UNE ZONE DE PAROI DANS UN ESPACE INTÉRIEUR D'UN OEIL ET DISPOSITIF OPTIQUE MUNI D'UN DISPOSITIF DE RETENUE ET D'UN IMPLANT OPTIQUE

(30) Priorität: 19.06.2018 DE 202018103444 U
(43) Veröffentlichungstag der Anmeldung: 28.04.2021
(73) Patentinhaber: Carriazo, Cesar C., Barranquilla (CO)
(72) Erfinder: Carriazo, Cesar C., Barranquilla (CO)
(74) Vertreter: Maiwald GmbH
(86) Internationale Anmeldenummer: PCT/IB2019/054730
(87) Internationale Veröffentlichungsnummer: WO 2019/243937

(56) Entgegenhaltungen:
- WO-A1-2014/137306
- WO-A1-2016/182520
- WO-A1-2017/116357
- WO-A1-2017/183359

## Beschreibung

Die Erfindung betrifft eine Haltevorrichtung zum Halten eines Optikimplantats an einem Wandbereich in einem Augeninnenraum eines Auges. Ein weiterer Aspekt der Erfindung betrifft eine Optikvorrichtung mit einer derartigen Haltevorrichtung und mit einem an der Haltevorrichtung gehaltenen Optikimplantat.

Aus dem Stand der Technik sind verschiedene Arten und Modelle von Optikvorrichtungen bekannt, welche als intraokulare Augenlinsen, auch Intraokularlinsen genannt, ausgebildet sein können. Die Intraokularlinse wird in der Regel im Rahmen einer Implantation in einem Kapselsack oder in einem Sulkus (skleraler Sulkus, scleral sulcus, sulcus sclerae) vernäht, insbesondere wenn die Intraokularlinse nicht ausreichend sicher im Auge, beispielsweise am Kapselsack, abgestützt werden kann. Eine Hauptursache für Schäden am Kapselsack stellt eine sogenannte Kapselruptur infolge einer chirurgischen Komplikation während einer Kataraktoperation dar, jedoch sind auch Krankheiten bzw. Situationen bekannt, durch welche eine Ablösung des Kapselsacks oder Subluxationen verursacht werden. Lediglich beispielhaft sind in diesem Zusammenhang Ablösungen bzw. Subluxationen infolge eines Marfan-Syndroms oder infolge von Traumata zu nennen. Im Allgemeinen bestehen Intraokularlinsen aus einer Optik, welche mit flexiblen Haptikelementen verbunden ist, sodass nicht nur ein minimalinvasives Einführen, beispielsweise in den Kapselsack, ermöglicht ist, sondern auch eine ausreichend sichere Zentrierung.

Die WO 98/10717 A1 beschreibt eine künstliche intraokulare Augenlinse mit einem verformbaren optischen Zonenbereich zur Implantation bei einer Kataraktoperation, die eine Linse und vier mit dieser verbundene winkelförmige elastische Haptikelemente aufweist, die peripher voneinander getrennt, alle in einer Drehrichtung derart angeordnet sind, dass in einem gefalteten Zustand die Haptikelemente ohne zusätzliche Manipulationen im Implantationsbereich zu liegen kommen. Die Haptikelemente weisen Einkerbungen oder Löcher auf, die dafür geeignet sind, eine Nahtfixation, zum Beispiel im Sulcus ciliaris, zu ermöglichen.

Die DE 199 25 636 B4 beschreibt eine Intraokularlinse für die Implantation in der Augen-Vorderkammer, bestehend aus einem mittig angeordneten Linsenkörper, der an seiner Peripherie mit Haptikelementen für die Fixierung an der Vorderseite der Iris versehen ist, wobei die Haptikelemente jeweils zwei sich von der Peripherie des Linsenkörpers nach außen erstreckende, zangenartige Arme aufweisen, zwischen deren Enden ein Spalt gebildet wird, in welchem die Iris einklemmbar ist.

Aus der WO 2014/137306 A1, der WO 2017/116357 A1, der WO 2016/182520 A1 sowie der WO 2017/183359 A1 sind jeweils Haltevorrichtungen zum Halten eines Optikimplantats an einem Wandbereich in einem Augeninnenraum eines Auges bekannt, wobei die Haltevorrichtungen jeweils zumindest eine Haltekomponente aufweist, mittels welcher eine Anpresskraft auf den Wandbereich ausübbar ist und dadurch die zumindest eine Haltekomponente an dem Wandbereich abstützbar ist, bekannt. Die Haltekomponente weist dabei einen Vorsprungbereich auf, von welchem sich zumindest ein Vorsprungelement weg erstreckt, welches dazu ausgebildet ist, bei Ausübung der Anpresskraft zumindest bereichsweise in ein Wandbereichsgewebe des Wandbereichs einzudringen und/oder punktuell Druck auf den Wandbereich auszuüben.

Aufgabe der vorliegenden Erfindung ist es, eine Haltevorrichtung sowie eine Optikvorrichtung der eingangs genannten Art zu schaffen, welche besonders aufwandsarm in einem Augeninnenraum festlegbar sind.

Diese Aufgabe wird durch eine Haltevorrichtung mit den Merkmalen des Anspruchs 1 sowie durch eine Optikvorrichtung mit den Merkmalen des Anspruchs 9gelöst. Vorteilhafte Ausgestaltungen sind in den Unteransprüchen beschrieben.

Ein erster Aspekt der Erfindung betrifft eine Haltevorrichtung zum Halten eines Optikimplantats an einem Wandbereich in einem Augeninnenraum eines Auges, umfassend zumindest eine Haltekomponente, mittels welcher eine Anpresskraft auf den Wandbereich ausübbar und dadurch die zumindest eine Haltekomponente an dem Wandbereich abstützbar ist. Das Optikimplantat kann beispielsweise als Intraokularlinse ausgebildet sein, durch welche Sehstörungen aufwandsarm ausgeglichen werden können. Des Weiteren kann das Optikimplantat als Prothese, also als künstliche Nachbildung zumindest eines Augenteilbereichs, ausgebildet sein, sodass anhand des Optikimplantats eine kosmetische Korrektur ermöglicht ist. Der Wandbereich kann den Augeninnenraum zumindest bereichsweise unmittelbar begrenzen. Der Wandbereich kann beispielsweise ein Sulkus (auch skleraler Sulkus genannt) oder ein Kapselsack des Auges sein. Sulkus bzw. Kapselsack sind im Vergleich zu anderen Augenbereichen besonders stabil und belastbar und damit besonders geeignet um die zumindest eine Haltekomponente unter Ausübung der Anpresskraft daran abzustützen. Die zumindest eine Haltekomponente kann zur unmittelbaren Abstützung an dem Wandbereich ausgebildet sein. Bei der unmittelbaren Abstützung kann in vorteilhafter Weise auf etwaige Zwischenelemente zwischen der Haltekomponente und dem Wandbereich verzichtet werden, wodurch eine besonders aufwandsarme Festlegung der Haltevorrichtung im Augeninnenraum ermöglicht ist.

Gemäß der Erfindung weist die zumindest eine Haltekomponente wenigstens einen Vorsprungbereich auf, von welchem sich zumindest ein Vorsprungelement weg erstreckt, welches dazu ausgebildet ist, bei Ausübung der Anpresskraft zumindest bereichsweise in ein Wandbereichsgewebe des Wandbereichs einzudringen und/oder punktuell Druck auf den Wandbereich auszuüben. Dies ist von Vorteil, da hierdurch ein besonders aufwandsarmes Festlegen der Haltevorrichtung an dem Wandbereich in dem Augeninnenraum des Auges ermöglicht ist. Durch das Vorsprungelement kann beispielsweise auf ein Vernähen, also auf eine Nahtfixation der Haltevorrichtung verzichtet werden, wodurch ein besonders aufwandsarmes Fixieren der Haltevorrichtung in dem Augeninnenraum ermöglicht ist. Das bereichsweise Eindringen in das Wandbereichsgewebe bzw. das punktuelle Ausüben des Drucks ermöglicht eine einfache, aufwandsarme Fixierung der Haltevorrichtung. Das bereichsweise Eindringen ist dabei von Vorteil, da hierdurch eine plastische Verformung des Wandbereichs sowie ein Formschluss zwischen der Haltekomponente und dem Wandbereich ausgebildet werden kann, wodurch ein besonders zuverlässiges Festlegen sowie eine Sicherung der Haltevorrichtung gegen Verdrehen (Verdrehsicherung) in dem Augeninnenraum ermöglicht ist.

Unter punktuellem Ausüben von Druck kann im Rahmen der vorliegenden Offenbarung ein Ausüben wenigstens einer Punktlast auf den Wandbereich verstanden werden. Dies ist von Vorteil, da durch die Punktlast eine besonders große Reibung zwischen der Haltevorrichtung und dem Wandbereich erzielt werden kann. Dadurch kann ein punktförmiges Eindrücken des Wandbereichs bewirkt und somit eine besonders vorteilhafte Lagesicherung der Haltekomponente erzielt werden.

Unter dem punktuellen Ausüben von Druck kann im Rahmen der vorliegenden Offenbarung auch ein Ausüben wenigstens einer Linienlast auf den Wandbereich verstanden werden. Dies ist von Vorteil, da durch das Ausüben der Linienlast eine linienförmige Druckverteilung auf den Wandbereich ermöglicht ist, wodurch ebenfalls eine besonders vorteilhafte Lagesicherung der Haltekomponente erzielt werden kann.

Das wenigstens eine Vorsprungelement kann vorzugsweise als Spitze oder als Haken ausgebildet sein. Dies ermöglicht ein einfaches Eindringen in das Wandbereichsgewebe des Wandbereichs und zusätzlich oder alternativ ein wirksames, punktuelles Ausüben des Drucks auf den Wandbereich.

Alternativ dazu kann das wenigstens eine Vorsprungelement auch bevorzugt als Stegelement ausgebildet sein. Das Stegelement kann in einer senkrecht zu dessen Haupterstreckungsrichtung verlaufenden Ebene einen dreieckigen Querschnitt aufweisen. Dies ermöglicht ebenfalls ein einfaches Eindringen in das Wandbereichsgewebe des Wandbereichs und zusätzlich oder alternativ ein wirksames, punktuelles Ausüben des Drucks auf den Wandbereich.

Die Haltevorrichtung kann allgemein faltbar ausgebildet sein um ein vereinfachtes Einführen der Haltevorrichtung in den Augeninnenraum zu ermöglichen. Nach dem Einführen in den Augeninnenraum kann die Haltevorrichtung entfaltet werden um die Haltevorrichtung in dem Augeninnenraum festzulegen.

Der Erfindung liegt die Erkenntnis zugrunde, dass es mit dem bereichsweisen Eindringen des zumindest einen Vorsprungelements in das Wandbereichsgewebe zwar zu minimalen Verletzungen an dem Wandbereichsgewebe kommen kann, jedoch derartige Verletzungen toleriert werden können, um die Haltevorrichtung zuverlässig und aufwandsarm festzulegen. Des Weiteren liegt die Erkenntnis zugrunde, dass auch durch das punktuelle Ausüben von Druck anhand des zumindest einen Vorsprungelements eine im Vergleich zu aus dem Stand der Technik bekannten Systemen (mit Intraokularlinsen) geringere Anpresskraft ausreichen kann, um ein zuverlässiges Festlegen zu erzielen, ohne dass hierfür eine Nahtfixation erforderlich wäre. Dies ist damit zu begründen, dass durch das punktuelle Ausüben von Druck ein punktuelles elastisches Eindrücken des Wandbereichs erzielt werden kann, wodurch - im Gegensatz zu einem flächigen Kontakt zwischen der Haltevorrichtung und dem Wandbereich - lokal eine besonders große Reibkraft bzw. Haltekraft zwischen dem Wandbereich und dem Vorsprungelement erzielt werden kann.

Gemäß der Erfindung umfasst die zumindest eine Haltekomponente wenigstens zwei Haptikelemente, mittels welchen die Anpresskraft auf den Wandbereich ausübbar ist, wobei der wenigstens eine Vorsprungbereich an zumindest einem Haptikelement der wenigstens zwei Haptikelemente angeordnet ist. Dies ist von Vorteil, da über die wenigstens zwei Haptikelemente eine besonders einfache Zentrierung der Haltekomponente ermöglicht ist. Hierzu können die wenigstens zwei Haptikelemente, beispielsweise in einer Längserstreckungsrichtung der Haltevorrichtung einander gegenüberliegend angeordnet sein, wodurch ein einfaches, mittiges Halten der Haltevorrichtung im Augeninnenraum ermöglicht ist. Der wenigstens eine Vorsprungbereich kann an einem distalen Ende des jeweiligen Haptikelements angeordnet sein.

Die zumindest eine Haltekomponente kann besonders bevorzugt genau zwei Haptikelemente umfassen. Dadurch können beim Festlegen der Haltekomponente, im Gegensatz zu aus dem Stand der Technik bekannten Systemen mit beispielsweise vier Haptikelementen, Spannungen am Auge besonders gering gehalten werden, wodurch eine unnötige Belastung des Auges vermieden werden kann. Sofern die Haltekomponente genau zwei Haptikelemente umfasst, so können diese zwei Haptikelemente besonders bevorzugt punktsymmetrisch zueinander ausgerichtet sein. Durch eine punktsymmetrische Ausrichtung kann eine besonders gleichmäßige, spannungsarme und damit gewebeschonende Belastung an dem Wandbereich erzielt werden. Insbesondere können durch die punktsymmetrische Ausrichtung etwaige Gewebedehnungen an dem Wandbereich zwischen den zwei Haptikelementen besonders gering gehalten werden.

Von Vorteil ist weiterhin, wenn sämtliche Haptikelemente jeweils einen Vorsprungbereich aufweisen, wodurch eine gleichzeitig besonders einfache und wirksame Verdrehsicherung der Haltevorrichtung ermöglicht ist.

Gemäß der Erfindung umfasst die Haltevorrichtung zumindest eine Aufnahmekomponente, welche mit den wenigstens zwei Haptikelementen verbunden ist und an welcher das Optikimplantat reversibel lösbar fixierbar ist. Dies ist von Vorteil, da das Optikimplantat hierdurch bei Bedarf besonders einfach zerstörungsfrei ausgetauscht werden kann, ohne die Haltevorrichtung hierzu ersetzen zu müssen. Die Haltevorrichtung kann somit dauerhaft im Augeninnenraum verbleiben, sodass ein einfacher Tausch des Optikimplantats durch einen besonders schonenden, minimalinvasiven, Eingriff ermöglicht ist. Die Aufnahmekomponente kann bevorzugt einen Halterahmen aufweisen, in welchem das Optikimplantat besonders ortsfest und sicher fixierbar ist.

Gemäß der Erfindung ist zudem die zumindest eine Aufnahmekomponente dazu ausgebildet, sich unter Ausübung der Anpresskraft zumindest bereichsweise von einer Nichtgebrauchsform, in welcher die Haltevorrichtung zumindest bereichsweise durch den Wandbereich begrenzten Augeninnenraum einführbar ist, in eine Aufnahmeform, in welcher das Optikimplantat an der zumindest einen Aufnahmekomponente fixierbar ist, aufzuweiten. Dies ist von Vorteil, da die Aufnahmekomponente somit in wenigstens einer Erstreckungsrichtung, beispielsweise in einer Quererstreckungsrichtung, in der Nichtgebrauchsform eine kleinere Abmessung aufweisen kann, als in der Aufnahmeform. Aufgrund der kleineren Abmessung in der Nichtgebrauchsform ist ein einfacheres Einführen der Haltevorrichtung in den Augeninnenraum ermöglicht. Dadurch, dass die Aufnahmekomponente in deren Aufnahmeform im Vergleich zur Nichtgebrauchsform aufgeweitet ist, kann das Optikimplantat besonders verliersicher an der Aufnahmekomponente (in deren Aufnahmeform) gehalten werden. Durch die Möglichkeit des Aufweitens von der Nichtgebrauchsform in die Aufnahmeform eignet sich die Haltevorrichtung besonders für Mikroinzisionen.

In einer weiteren vorteilhaften Weiterbildung der Erfindung ist zumindest ein Haptikelement der wenigstens zwei Haptikelemente unter Ausübung der Anpresskraft elastisch verformbar. Dies ist von Vorteil, da die wenigstens zwei Haptikelemente somit vorgespannt sein können, um die Anpresskraft auf den Wandbereich auszuüben. Aufgrund der elastischen Verformung der Haptikelemente ist eine dauerhafte Ausübung der Anpresskraft anhand der Haptikelemente ermöglicht, wodurch die Haltevorrichtung entsprechend dauerhaft haltbar in dem Augeninnenraum festlegbar ist.

In einer weiteren vorteilhaften Weiterbildung der Erfindung ist zumindest ein Haptikelement der wenigstens zwei Haptikelemente bogenförmig ausgebildet. Dies ist von Vorteil, da durch ein bogenförmiges Ausbilden nicht nur eine besonders einfache elastische Verformbarkeit ermöglicht ist, sondern auch ein gewebeschonendes Abfedern, beispielsweise von Stoßbelastungen bzw. Erschütterungen, ermöglicht ist. Infolge des Ausübens der Anpresskraft kann sich ein Krümmungsradius des bogenförmig ausgebildeten Haptikelements verringern, wodurch ein besonders gleichmäßiger Kraftfluss ohne etwaige Spannungsspitzen im Haptikelement erzielt werden kann.

In einer weiteren vorteilhaften Weiterbildung der Erfindung ist zumindest ein Haptikelement der wenigstens zwei Haptikelemente an einem ersten Endbereich des zumindest einen Haptikelements mit der zumindest einen Aufnahmekomponente verbunden und an einem dem ersten Endbereich gegenüberliegenden, zweiten Endbereich ist der wenigstens eine Vorsprungbereich angeordnet. Dies ist von Vorteil, da das zumindest eine Haptikelement somit eine besonders hohe Funktionalität aufweist. Zum einen dient das zumindest eine Haptikelement somit zum Halten der Aufnahmekomponente und zum anderen zum Abstützen an dem Wandbereich. Aufgrund dieser hohen Funktionalität des zumindest einen Haptikelements ist ein besonders einfacher und kompakter Aufbau der Haltevorrichtung sowie deren aufwandsarmes Festlegen in dem Augeninnenraum ohne Nahtfixation ermöglicht.

In einer weiteren vorteilhaften Weiterbildung der Erfindung sind einander gegenüberliegende Endbereiche zumindest eines Haptikelements der wenigstens zwei Haptikelemente mit der Aufnahmekomponente verbunden und zumindest ein zwischen den jeweiligen Endbereichen angeordneter Mittelbereich des zumindest einen Haptikelements ist von der Aufnahmekomponente beabstandet, wobei der wenigstens eine Vorsprungbereich an dem zumindest einen Mittelbereich angeordnet ist. Dies ist von Vorteil, da durch die Verbindung des zumindest einen Haptikelements an dessen einander gegenüberliegenden Endbereichen eine besonders stabile Fixierung zwischen dem Haptikelement und der Aufnahmekomponente gebildet ist. Dadurch, dass der Vorsprungbereich an dem zwischen den Endbereichen angeordnete Mittelbereich angeordnet ist, ist eine besonders gleichmäßige Lastverteilung der Anpresskraft, bzw. einer aus der Anpresskraft resultierenden Kraft über die Endbereiche ermöglicht, wodurch beispielsweise Spannungen oder Torsionen innerhalb der Aufnahmekomponente gering gehalten werden können.

In einer weiteren vorteilhaften Weiterbildung der Erfindung ist die zumindest eine Aufnahmekomponente unter Ausübung der Anpresskraft mittels der wenigstens zwei Haptikelemente elastisch verformbar. Dies ist von Vorteil, da der Aufnahmekomponente somit eine Doppelfunktion zukommt. Die Aufnahmekomponente dient somit nicht nur zum reversibel lösbaren Fixieren des Optikimplantats, sondern trägt auch zur Ausübung der Anpresskraft bei.

In einer weiteren vorteilhaften Weiterbildung der Erfindung umfasst die zumindest eine Aufnahmekomponente wenigstens einen Klemmbereich, an welchem das Optikimplantat einklemmbar und dadurch lösbar fixierbar ist. Dies ist von Vorteil, da durch den Klemmbereich ein Einklemmen des Optikimplantats und damit eine besonders einfache reversibel lösbare Fixierung des Optikimplantats an der Aufnahmekomponente ermöglicht ist.

In einer weiteren vorteilhaften Weiterbildung der Erfindung ist die Haltevorrichtung einteilig ausgebildet. Dies ist von Vorteil, da hierdurch eine besonders aufwandsarme Handhabung der Haltevorrichtung bei deren Festlegen im Augeninnenraum erfolgen kann.

Ein zweiter Aspekt der Erfindung betrifft eine Optikvorrichtung mit einer Haltevorrichtung gemäß dem ersten Aspekt der Erfindung und mit einem an der Haltevorrichtung gehaltenen Optikimplantat. Eine derartige Optikvorrichtung ist besonders aufwandsarm in einem Augeninnenraum festlegbar. Die im Zusammenhang mit der Haltevorrichtung gemäß dem ersten Aspekt der Erfindung vorgestellten Merkmale sowie deren Vorteile gelten entsprechend für die Optikvorrichtung gemäß dem zweiten Aspekt der Erfindung und umgekehrt.

Das Optikimplantat kann allgemein faltbar ausgebildet sein um ein vereinfachtes Einführen des Optikimplantats in den Augeninnenraum zu ermöglichen. Nach dem Einführen in den Augeninnenraum kann das Optikimplantat entfaltet werden um das Optikimplantat in dem Augeninnenraum festzulegen.

In einer weiteren vorteilhaften Weiterbildung der Erfindung ist die Optikvorrichtung einteilig ausgebildet. Dies ist von Vorteil, da hierdurch eine besonders aufwandsarme Handhabung der Optikvorrichtung bei deren Festlegen im Augeninnenraum erfolgen kann.

In einer weiteren vorteilhaften Weiterbildung der Erfindung ist das Optikimplantat reversibel lösbar an der Haltevorrichtung fixiert. Dies ist von Vorteil, da das Optikimplantat hierdurch bei Bedarf besonders einfach zerstörungsfrei ausgetauscht werden kann, ohne die Haltevorrichtung ersetzen zu müssen. Die Haltevorrichtung kann dabei im Augeninnenraum verbleiben, sodass eine besonders kleine Öffnung, bzw. ein besonders kleiner Schnitt am Auge zum Austauschen des Optikimplantats ausreicht.

Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar.

Weitere Vorteile, Merkmale und Einzelheiten der Erfindung ergeben sich aus den Ansprüchen, der nachfolgenden Beschreibung bevorzugter Ausführungsformen sowie anhand der Zeichnungen.

Im Folgenden ist die Erfindung noch einmal anhand eines konkreten Ausführungsbeispiels erläutert. Hierzu zeigt:
- Fig. 1a-b: jeweils schematische Schnittdarstellungen eines Teilbereichs eines Auges sowie jeweilige Draufsichten auf eine Optikvorrichtung, welche an einem, den Augeninnenraum begrenzenden Wandbereich des Auges abstützbar ist, wobei die Optikvorrichtung in Fig. 1a von dem Wandbereich gelöst und in Fig. 1b an dem Wandbereich abgestützt ist;
- Fig. 2a-b: jeweils weitere schematische Schnittdarstellungen des Teilbereichs des Auges sowie jeweilige Draufsichten auf eine Haltevorrichtung zum Halten eines Optikimplantats, wobei die Haltevorrichtung in Fig. 2a von dem Wandbereich gelöst und in Fig. 2b an dem Wandbereich abgestützt ist;
- Fig. 3a-b: jeweilige weitere schematische Schnittdarstellungen des Teilbereichs des Auges sowie jeweilige Draufsichten auf eine Variante der Haltevorrichtung zum Halten des Optikimplantats, wobei die Haltevorrichtung in Fig. 3a von dem Wandbereich gelöst und in Fig. 3b an dem Wandbereich abgestützt ist;
- Fig. 4a: eine schematische Draufsicht auf eine weitere Variante der Haltevorrichtung zum Halten des Optikimplantats, wobei die Haltevorrichtung in einer Nichtgebrauchsform gezeigt ist;
- Fig. 4b: eine schematische Seitenansicht der in Fig. 4a gezeigten Variante der Haltevorrichtung in deren Nichtgebrauchsform;
- Fig. 4c: eine schematische Draufsicht auf die weitere Variante der Haltevorrichtung zum Halten des Optikimplantats, wobei die Haltevorrichtung in einer Aufnahmeform gezeigt ist, in welcher die Haltevorrichtung im Vergleich zur Nichtgebrauchsform aufgeweitet und an dem Wandbereich abgestützt ist; und
- Fig. 4d: eine schematische Draufsicht auf die weiterer Variante der Haltevorrichtung in deren Aufnahmeform, wobei das Optikimplantat reversibel lösbar an der Haltevorrichtung fixiert ist.

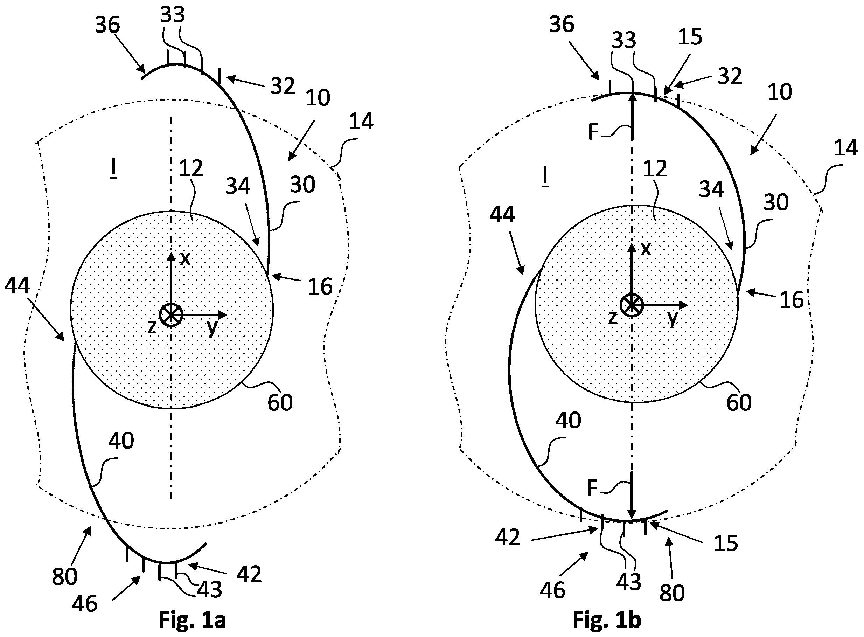

Fig. 1a und Fig. 1b zeigen jeweils eine Optikvorrichtung 80, welche eine Haltevorrichtung 10 und ein an der Haltevorrichtung 10 gehaltenes Optikimplantat 12 umfasst. Das Optikimplantat 12 ist vorliegend als Intraokularlinse ausgebildet.

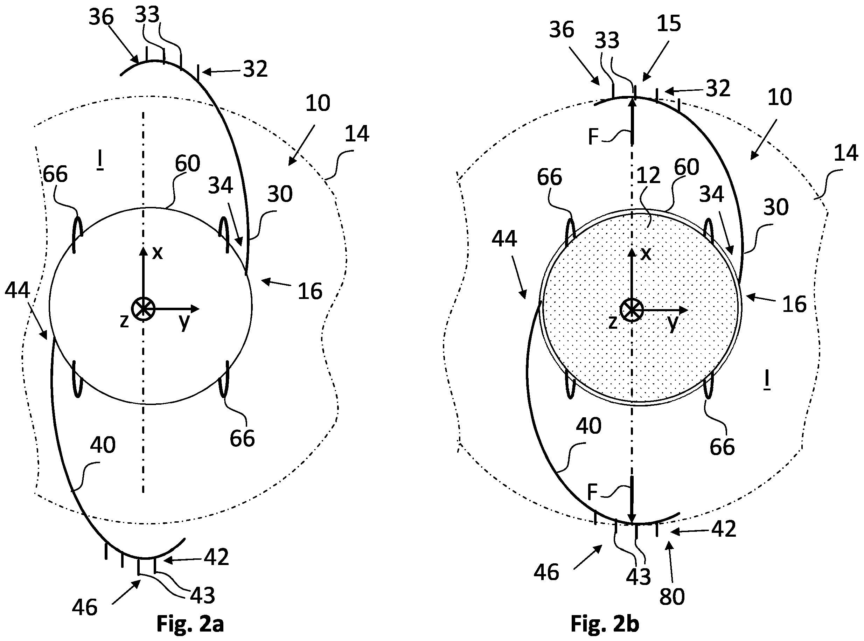

Die Optikvorrichtung 80 kann allgemein einteilig ausgebildet sein. Das Optikimplantat 12 kann alternativ dazu auch reversibel lösbar an der Haltevorrichtung 10 fixiert werden, sodass die Haltevorrichtung 10 unabhängig von dem Optikimplantat 12 bereitgestellt bzw. in einem Augeninnenraum I eines Auges festgelegt werden kann, wie anhand von Fig. 2a bis Fig. 4c erkennbar ist. Die Haltevorrichtung 10 kann allgemein einteilig ausgebildet sein.

In den Fig. 1a bis Fig. 4c sind jeweils auf die Optikvorrichtung 80 bzw. auf die Haltevorrichtung 10 bezogene Koordinatensysteme angegeben, welche durch eine Längserstreckungsrichtung x, durch eine Quererstreckungsrichtung y sowie durch eine Hocherstreckungsrichtung z der Optikvorrichtung 80 bzw. der Haltevorrichtung 10 definiert sind.

Fig. 2a bis Fig. 4c zeigen jeweils die Haltevorrichtung 10 zum Halten des Optikimplantats 12 an einem Wandbereich 14 des Auges in dem Augeninnenraum I. Die Haltevorrichtung 10 umfasst allgemein eine Haltekomponente 16, mittels welcher eine Anpresskraft F auf den Wandbereich 14 ausgeübt und dadurch die Haltekomponente 16 an dem Wandbereich 14 abgestützt werden kann. Die Anpresskraft F wird allgemein anhand der Haltekomponente 16 auf den Wandbereich 14 ausgeübt, wenn die Haltekomponente 16 in einem (gegenüber dem Wandbereich 14) verspannten Zustand ist, also sobald die Haltevorrichtung 10 in den Augeninnenraum I eingeführt und die Haltekomponente 16 in Stützanlage mit dem Wandbereich 14 ist, wie in Fig. 1b, Fig. 2b, Fig. 3b, Fig. 4c und Fig. 4d erkennbar ist.

In Fig. 1a, Fig. 2a, Fig. 3a, Fig. 4a und Fig. 4b ist die Haltekomponente 16 in einem entspannten Zustand außerhalb des Augeninnenraums I gezeigt, wobei zumindest in Fig. 1a, Fig. 2a und Fig. 3a, in welchen die Haltevorrichtung 10 bzw. die Optikvorrichtung 80 noch nicht in den Augeninnenraum I eingeführt sind, erkennbar ist, dass die Haltekomponente 16 im entspannten Zustand wenigstens in der Längserstreckungsrichtung x eine größere Erstreckung aufweist, als der Wandbereich 14. Im entspannten Zustand überragt die Haltekomponente 16 dabei einen Innendurchmesser des Wandbereichs 14 in der Längserstreckungsrichtung x. Dementsprechend überragen zwei Haptikelemente 30, 40 der Haltekomponente 16 in deren entspanntem Zustand den Wandbereich 14 in der Längserstreckungsrichtung x, wie zumindest in Fig. 1a, Fig. 2a und Fig. 3a erkennbar ist.

Die Haltekomponente 16 ist allgemein dazu ausgebildet, bei Ausübung der Anpresskraft F zumindest bereichsweise in ein Wandbereichsgewebe 15 des Wandbereichs 14 einzudringen und zusätzlich oder alternativ punktuell Druck auf den Wandbereich 14 auszuüben. Hierzu weist die Haltekomponente 60 jeweilige Vorsprungbereiche 32, 42 auf, von welchen sich jeweils mehrere Vorsprungelemente 33, 43 weg erstrecken, wie aus der Zusammenschau von Fig. 1a bis Fig. 4c erkennbar ist. Die Vorsprungelemente 33 sind allgemein dem Vorsprungbereich 32 zugeordnet und an diesem angeordnet. Die Vorsprungelemente 43 sind allgemein dem Vorsprungbereich 42 zugeordnet und an diesem angeordnet. Die Vorsprungelemente 33, 43 sind vorliegend als jeweilige Spitzen ausgebildet.

Zumindest einige der Vorsprungelemente 33, 43 können auch als jeweilige Stegelemente ausgebildet sein, was vorliegend jedoch nicht weiter dargestellt ist. Die Stegelemente können in einer senkrecht zu deren Haupterstreckungsrichtung verlaufenden Ebene einen dreieckigen Querschnitt aufweisen. Diese Ebene kann durch die Längserstreckungsrichtung x und durch die Quererstreckungsrichtung y aufgespannt sein.

Die Vorsprungbereiche 32, 42 sind vorliegend an den Haptikelementen 30, 40, mittels welchen die Anpresskraft F auf den Wandbereich 14 ausübbar ist und welche jeweils einen der Vorsprungbereiche 32, 42 aufweisen angeordnet. Der Vorsprungbereich 32 ist dabei dem Haptikelement 30 zugeordnet, wohingegen der Vorsprungbereich 42 dem Haptikelement 40 zugeordnet ist.

Die Haptikelemente 30, 40 sind allgemein unter Ausübung der Anpresskraft F elastisch verformbar und bogenförmig ausgebildet.

Die Haltevorrichtung 10 kann allgemein eine Aufnahmekomponente 60 umfassen, welche mit den Haptikelementen 30, 40 verbunden ist und an welcher das Optikimplantat 12 reversibel lösbar fixierbar ist, wie insbesondere in Fig. 2a bis Fig. 4d erkennbar ist.

Die Haptikelemente 30, 40 können an einem jeweiligen ersten Endbereich 34, 44 mit der Aufnahmekomponente 60 verbunden sein und die jeweiligen Vorsprungbereiche 32, 42 können an einem jeweiligen, dem ersten Endbereich 34, 44 gegenüberliegenden, zweiten Endbereich 36, 46 angeordnet sein, wie beispielsweise in Fig. 1a, Fig. 1b, Fig. 2a, Fig. 2b erkennbar ist. Der erste Endbereich 34 und der zweite Endbereich 36 sind dem ersten Haptikelement 30 zugeordnet, wohingegen der erste Endbereich 44 und der zweite Endbereich 46 dem zweiten Haptikelement 40 zugeordnet sind.

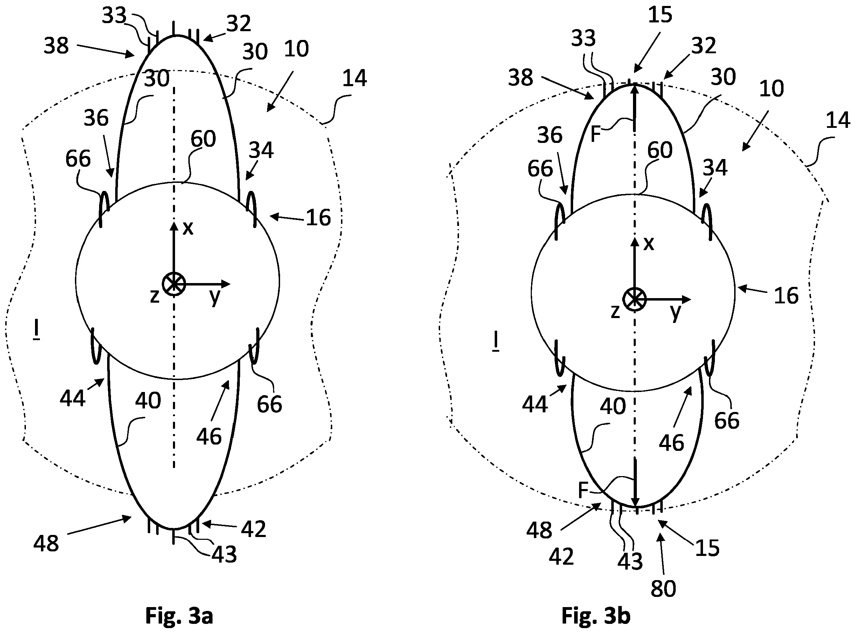

Fig. 3a bis Fig. 4d zeigen, dass die einander gegenüberliegenden Endbereiche 34, 36, des ersten Haptikelements 30 sowie die einander gegenüberliegenden Endbereiche 44, 46 des zweiten Haptikelements 40 auch mit der Aufnahmekomponente 60 verbunden sein können. Dadurch sind die Haptikelemente 30, 40 sozusagen jeweils beidseitig mit der Aufnahmekomponente 60 verbunden, wodurch die Haptikelemente 30, 40 keine freien Enden aufweisen. Ein zwischen den jeweiligen Endbereichen 34, 36 angeordneter Mittelbereich 38 des ersten Haptikelements 30 und ein zwischen den jeweiligen Endbereichen 44, 46 des zweiten Haptikelements 40 angeordneter Mittelbereich 48 können dabei von der Aufnahmekomponente 60 beabstandet sein. Die jeweiligen Mittelbereiche 38, 48 erstrecken sich vorliegend jeweils in Längserstreckungsrichtung x von der Aufnahmekomponente 60 weg.. Der Vorsprungbereich 32 kann an dem Mittelbereich 38 angeordnet sein, wohingegen der Vorsprungbereich 42 an dem Mittelbereich 48 angeordnet sein kann. Mit anderen Worten kann der Vorsprungbereich 32 dem Mittelbereich 38 des ersten Haptikelements 30 und der Vorsprungbereich 42 dem Mittelbereich 48 des zweiten Haptikelements 40 zugeordnet sein.

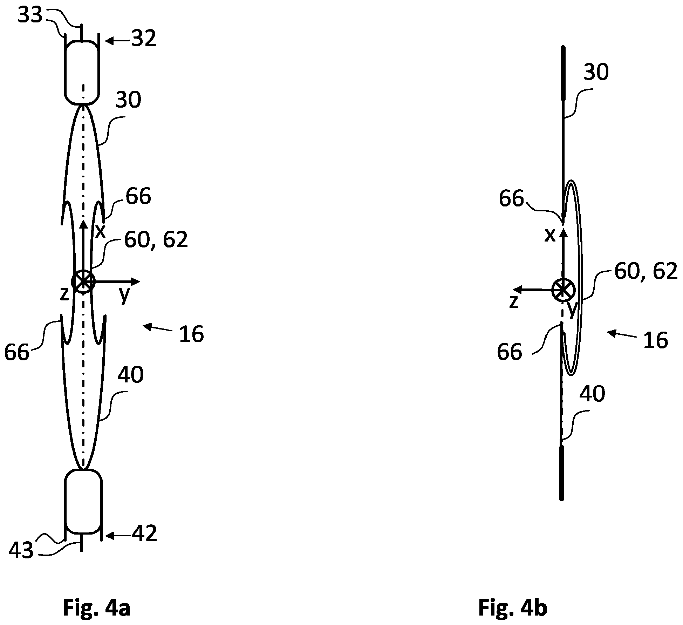

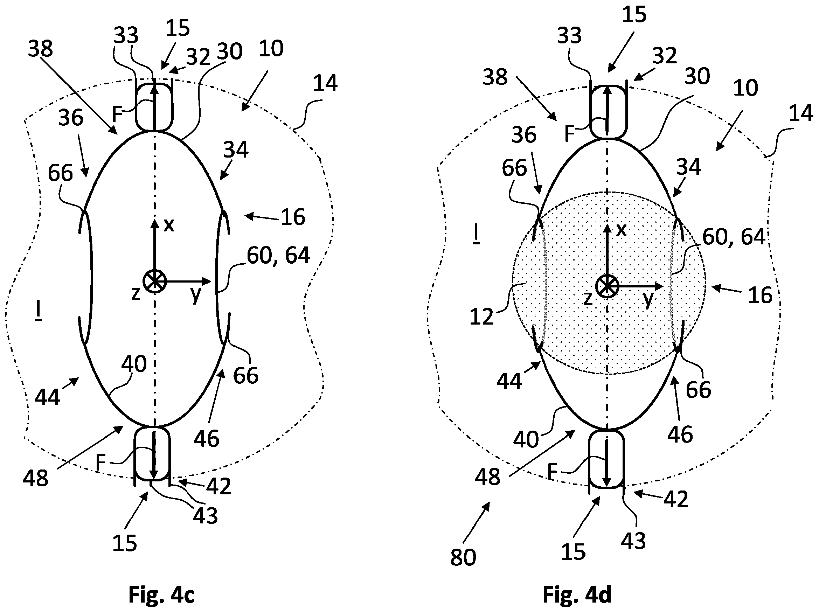

Aus der Zusammenschau von Fig. 4a bis Fig. 4d ist erkennbar, dass die Aufnahmekomponente 60 unter Ausübung der Anpresskraft F mittels der Haptikelemente 30, 40 verformbar sein kann. Die Aufnahmekomponente 60 kann dazu ausgebildet sein, sich unter Ausübung der Anpresskraft F zumindest bereichsweise von einer, in Fig. 4a und Fig. 4b gezeigten Nichtgebrauchsform 62, in welcher die Haltevorrichtung 10 besonders einfach in den zumindest bereichsweise durch den Wandbereich 14 begrenzten Augeninnenraum I einführbar ist, in eine, in Fig. 4c und Fig. 4d gezeigte Aufnahmeform 64, in welcher das Optikimplantat 12 an der zumindest einen Aufnahmekomponente 60 reversibel lösbar fixierbar ist, aufzuweiten. Besonders aus der Zusammenschau von Fig. 4a mit Fig. 4c ist erkennbar, dass ein entsprechendes Aufweiten der Aufnahmekomponente 60 bei deren Verformung von der Nichtgebrauchsform 62 in die Aufnahmeform 64 in Quererstreckungsrichtung y erfolgen kann. In diesem Fall kann ein Einführen der Haltevorrichtung 10 in den Augeninnenraum I in Längserstreckungsrichtung x durch eine besonders kleine, hier nicht weiter gezeigte Öffnung im Wandbereich 14 erfolgen, zumal die Aufnahmekomponente 60 in der in Fig. 4a besonders deutlich erkennbaren Nichtgebrauchsform 62 in Quererstreckungsrichtung y eine kleinere Erstreckung aufweist, also vorliegend schmaler ist, als in der in Fig. 4c besonders deutlich erkennbaren Aufnahmeform 64.

Fig. 2a bis Fig. 4d zeigen, dass die Aufnahmekomponente 60, welcher allgemein als Rahmen oder als Rahmenelement ausgebildet sein kann, mehrere Klemmbereiche 66 umfassen kann, an welchem das Optikimplantat 12 einklemmbar und dadurch lösbar fixierbar ist. Die Klemmbereiche 66 können als jeweilige Haltehaken ausgebildet sein, an welchem das Optikimplantat 12 eingeklemmt werden kann. Hierzu können die Klemmbereiche 66 das Optikimplantat 12 jeweils zumindest bereichsweise umgreifen.

Um eine besonders einfache, aufwandsarme und vor allem statisch bestimmte Lagerung des Optikimplantats 12 an der Aufnahmekomponente 60 zu ermöglichen, kann die Aufnahmekomponente 60 genau drei Klemmbereiche 66 umfassen, wenngleich vorliegend vier Klemmbereich 66 gezeigt sind. Die genau drei Klemmbereiche 66 können regelmäßig in einer durch die Längserstreckungsrichtung x und die Quererstreckungsrichtung y aufgespannten Ebene bzw. einer zu dieser parallelen Ebene angeordnet sein. Dabei können die Klemmbereiche 66 einen Winkelabstand von 120° zueinander aufweisen, wodurch eine besonders genaue Ausrichtung des Optikimplantats 12 bei dessen Fixierung mittels der Klemmbereiche 66 ermöglicht ist.

Allgemein ist durch die Haltevorrichtung 10 ein Selbstfixierungssystem zum Halten des Optikimplantats 12 bereitgestellt. Das Optikimplantat 12 kann allgemein als Linse, insbesondere als Intraokularlinse, ausgebildet sein. Die Haltevorrichtung 10 ist dazu ausgebildet sich unter Ausübung der Anpresskraft F an dem Wandbereich 14 abzustützen und ist dadurch gegenüber Drehbewegungen innerhalb des Augeninnenraums I gesichert. Die Haltevorrichtung 10 kann sich mit den jeweiligen Vorsprungelementen 33, 43 im Wandbereich 14 festsetzen und sich dabei beispielsweise im Wandbereich 14 verkrallen, wodurch eine besonders haltbare Befestigung gegeben ist.

Die Haltevorrichtung 10 bzw. die Optikvorrichtung 80 weisen in deren jeweiligen, entspannten Zustand, also vor deren Einführen in den Augeninnenraum I bzw. vor deren Festlegen am Wandbereich 14 in zumindest einer Erstreckungsrichtung, beispielsweise in der Längserstreckungsrichtung x, eine größere Erstreckung auf, als der Wandbereich 14, welcher beispielsweise ein Kapselsack, bzw. ein Sulkus (sulcus sklerae) des Auges sein kann. Dies ermöglicht es, dass die Haltevorrichtung 10 bzw. die Optikvorrichtung 80 gegenüber dem Wandbereich 14 verspannt werden können und dadurch die Anpresskraft F auf den Wandbereich 14 ausgeübt werden kann. Die Haltevorrichtung 10 ermöglicht es, dass von einem Chirurgen vorgesehene Optikimplantat 12 lagegesichert in dem Augeninnenraum I und abgestützt an dem Wandbereich 14 zu halten.

Sowohl die Haltevorrichtung als auch die Optikvorrichtung 80 können einem jeweiligen, gefalteten Zustand in das Auge und damit in den Augeninnenraum I anhand eines Injektors eingeführt werden, ohne hierfür eine große Öffnung in Form eines Schnittes an dem Wandbereich 14 vornehmen zu müssen.

## Patentansprüche

1. Haltevorrichtung (10) zum Halten eines Optikimplantats (12) an einem Wandbereich (14) in einem Augeninnenraum (I) eines Auges, umfassend zumindest eine Haltekomponente (16), mittels welcher eine Anpresskraft (F) auf den Wandbereich (14) ausübbar und dadurch die zumindest eine Haltekomponente (16) an dem Wandbereich (14) abstützbar ist, wobei die zumindest eine Haltekomponente (16) wenigstens einen Vorsprungbereich (32, 42) aufweist, von welchem sich zumindest ein Vorsprungelement (33, 43) weg erstreckt, welches dazu ausgebildet ist, bei Ausübung der Anpresskraft (F) zumindest bereichsweise in ein Wandbereichsgewebe (15) des Wandbereichs (14) einzudringen und/oder punktuell Druck auf den Wandbereich (14) auszuüben und wobei die zumindest eine Haltekomponente (16) wenigstens zwei Haptikelemente (30, 40) umfasst, mittels welchen die Anpresskraft (F) auf den Wandbereich (14) ausübbar ist, wobei der wenigstens eine Vorsprungbereich (32, 42) an zumindest einem Haptikelement der wenigstens zwei Haptikelemente (30, 40) angeordnet ist und die Haltevorrichtung (10) zumindest eine Aufnahmekomponente (60) umfasst, welche mit den wenigstens zwei Haptikelementen (30, 40) verbunden ist und an welcher das Optikimplantat (12) reversibel lösbar fixierbar ist, **dadurch gekennzeichnet, dass** die zumindest eine Aufnahmekomponente (60) dazu ausgebildet ist, sich unter Ausübung der Anpresskraft (F) zumindest bereichsweise von einer Nichtgebrauchsform (62), in welcher die Haltevorrichtung (10) in den zumindest bereichsweise durch den Wandbereich (14) begrenzten Augeninnenraum (I) einführbar ist, in eine Aufnahmeform (64), in welcher das Optikimplantat (12) an der zumindest einen Aufnahmekomponente (60) reversibel lösbar fixierbar ist, aufzuweiten.

2. Haltevorrichtung (10) nach Anspruch 1,
**dadurch gekennzeichnet, dass**
zumindest ein Haptikelement der wenigstens zwei Haptikelemente (30, 40) unter Ausübung der Anpresskraft (F) elastisch verformbar ist.

3. Haltevorrichtung (10) nach Anspruch 1 oder 2,
**dadurch gekennzeichnet, dass**
zumindest ein Haptikelement der wenigstens zwei Haptikelemente (30, 40) bogenförmig ausgebildet ist.

4. Haltevorrichtung nach Anspruch 1,
**dadurch gekennzeichnet, dass**
zumindest ein Haptikelement der wenigstens zwei Haptikelemente (30, 40) an einem ersten Endbereich (34, 44) des zumindest einen Haptikelements mit der zumindest eine Aufnahmekomponente (60) verbunden ist und an einem dem ersten Endbereich (34, 44) gegenüberliegenden, zweiten Endbereich (36, 46) der wenigstens eine Vorsprungbereich (32, 42) angeordnet ist.

5. Haltevorrichtung (10) nach Anspruch 1,
**dadurch gekennzeichnet, dass**
einander gegenüberliegende Endbereiche (34, 36, 44, 46) zumindest eines Haptikelements der wenigstens zwei Haptikelemente (30, 40) mit der Aufnahmekomponente (60) verbunden sind und zumindest ein zwischen den jeweiligen Endbereichen (34, 36, 44, 46) angeordneter Mittelbereich (38, 48) des zumindest einen Haptikelements von der Aufnahmekomponente (60) beabstandet ist, wobei der wenigstens eine Vorsprungbereich (32, 42) an dem zumindest einen Mittelbereich (38, 48) angeordnet ist.

6. Haltevorrichtung (10) nach einem der Ansprüche 1 bis 5,
**dadurch gekennzeichnet, dass**
die zumindest eine Aufnahmekomponente (60) unter Ausübung der Anpresskraft (F) mittels der wenigstens zwei Haptikelemente (30, 40) elastisch verformbar ist.

7. Haltevorrichtung (10) nach einem der Ansprüche 1 bis 6,
**dadurch gekennzeichnet, dass**
die zumindest eine Aufnahmekomponente (60) wenigstens einen Klemmbereich (66) umfasst, an welchem das Optikimplantat (12) einklemmbar und dadurch lösbar fixierbar ist.

8. Haltevorrichtung (10) nach einem der vorhergehenden Ansprüche,
**dadurch gekennzeichnet, dass**
die Haltevorrichtung (10) einteilig ausgebildet ist.

9. Optikvorrichtung (80) mit einer Haltevorrichtung (10) nach einem der Ansprüche 1 bis 8 und mit einem an der Haltevorrichtung (10) gehaltenen Optikimplantat (12).

10. Optikvorrichtung (80) nach Anspruch 9,
**dadurch gekennzeichnet, dass**
die Optikvorrichtung (80) einteilig ausgebildet ist.

11. Optikvorrichtung (80) nach Anspruch 9,
**dadurch gekennzeichnet, dass**
das Optikimplantat (12) reversibel lösbar an der Haltevorrichtung (10) fixiert ist.

## Claims

1. Holding device (10) for holding an optical implant (12) on a wall area (14) in an eye interior (I) of an eye, comprising at least one retaining component (16), by which a contact pressure (F) can be exerted on the wall area (14) and, as a result thereof, thereby the at least one retaining component (16) can be supported on the wall area (14), wherein the at least one retaining component (16) has at least one protrusion region (32, 42), from which at least one protrusion element (33, 43) extends away, which is designed to, when the contact pressure force (F) is exerted, penetrate at least regionally into a wall area tissue (15) of the wall area (14) and/or to exert punctiform pressure on the wall area (14) and wherein said at least one retaining component (16) comprises at least two haptic elements (30, 40) by which the contact pressure (F) can be exerted on the wall area (14), wherein the at least one protrusion region (32, 42) is arranged on at least one haptic element of the at least two haptic elements (30, 40) and the holding device (10) comprises at least one receiving component (60) which is connected to the at least two haptic elements (30, 40) and to which the optical implant (12) can be reversibly releasably fixed, **characterized in that** said at least one receiving component (60) is designed to expand, under the exertion of the contact pressure (F), at least regionally from a non-use shape (62), in which the holding device (10) can be introduced into the eye interior (I) bounded at least regionally by the wall area (14), into a receiving shape (64), in which the optical implant (12) can be fixed reversibly detachably to the at least one receiving component (60).

2. Holding device (10) according to claim 1,
**characterized in that**
at least one haptic element of the at least two haptic elements (30, 40) is elastically deformable under the exertion of the contact pressure (F).

3. Holding device (10) according to claim 1 or 2,
**characterized in that**
at least one haptic element of the at least two haptic elements (30, 40) is configured to be arcuate.

4. Holding device (10) according to claim 1,
**characterized in that**
at least one haptic element of the at least two haptic elements (30, 40) is connected to the at least one receiving component (60) at a first end region (34, 44) of the at least one haptic element, and the at least one protrusion region (32, 42) is arranged at a second end region (36, 46) opposite the first end region (34, 44).

5. Holding device (10) according to claim 1,
**characterized in that**
opposite end regions (34, 36, 44, 46) of at least one haptic element of the at least two haptic elements (30, 40) are connected to the receiving component (60), and at least one central region (38, 48) of the at least one haptic element arranged between the respective end regions (34, 36, 44, 46) is spaced apart from the receiving component (60), the at least one protrusion region (32, 42) being arranged on the at least one central region (38, 48).

6. Holding device (10) according to any one of claims 1 to 5,
**characterized in that**
the at least one receiving component (60) is elastically deformable under the exertion of the contact pressure (F) by the at least two haptic elements (30, 40).

7. Holding device (10) according to any one of claims 1 to 6,
**characterized in that**
the at least one receiving component (60) comprises at least one clamping region (66) to which the optical implant (12) can be fixed reversibly detachably.

8. Holding device (10) according to any one of the preceding claims,
**characterized in that**
the holding device (10) is formed in one piece.

9. Optical device (80) comprising a holding device (10) according to any one of claims 1 to 8 and comprising an optical implant (12) held on the holding device (10).

10. Optical device (80) according to claim 9,
**characterized in that**
the optical device (80) is formed in one piece.

11. Optical device (80) according to claim 92,
**characterized in that**
the optical implant (12) is reversibly detachably fixed to the holding device (10).

## Revendications

1. Dispositif de retenue (10) pour maintenir un implant optique (12) sur une zone de paroi (14) dans un espace intérieur oculaire (I) d'un oeil, comprenant au moins un composant de retenue (16) au moyen duquel une force de pression (F) peut être exercée sur la zone de paroi (14) et le composant de retenue (16) peut ainsi être en appui sur la zone de paroi (14), le au moins un composant de retenue (16) comportant au moins une zone de saillie (32, 42) à partir de laquelle s'étend au moins un élément de saillie (33, 43) en s'éloignant de celle-ci, qui est configuré pour, lorsque la force de pression (F) est appliquée, pénétrer au moins dans certaines zones d'un tissu de zone de paroi (15) de la zone de paroi (14) et/ou pour exercer ponctuellement une pression sur la zone de paroi (14), et le au moins un composant de retenue (16) comprenant au moins deux éléments haptiques (30, 40) au moyen desquels la force de pression (F) peut être exercée sur la zone de paroi (14), la au moins une zone de saillie (32, 42) étant disposée sur au moins un élément haptique des au moins deux éléments haptiques (30, 40) et le dispositif de retenue (10) comprenant au moins un composant de réception (60) qui est relié aux au moins deux éléments haptiques (30, 40) et sur lequel l'implant optique (12) peut être fixé de manière réversiblement détachable, **caractérisé en ce que** le au moins un composant de réception (60) est configuré pour s'élargir, sous l'action de la force de pression (F), au moins dans certaines zones, en passant d'une forme non utilisée (62) dans laquelle le dispositif de retenue (10) peut être introduit dans l'espace intérieur oculaire (I) délimité, au moins dans certaines zones, par la zone de paroi (14), à une forme de réception (64) dans laquelle l'implant optique (12) peut être fixé de manière réversiblement détachable au au moins un composant de réception (60).

2. Dispositif de retenue (10) selon la revendication 1,
**caractérisé en ce que**
au moins un élément haptique parmi les au moins deux éléments haptiques (30, 40) peut être déformé élastiquement sous l'action de la force de pression (F).

3. Dispositif de retenue (10) selon la revendication 1 ou 2,
**caractérisé en ce que**
au moins un élément haptique parmi les au moins deux éléments haptiques (30, 40) est réalisé en forme d'arc.

4. Dispositif de retenue selon la revendication 1,
**caractérisé en ce que**
au moins un élément haptique parmi les au moins deux éléments haptiques (30, 40) est relié au au moins un composant de réception (60) sur une première zone d'extrémité (34, 44) du au moins un élément haptique et la au moins une zone de saillie (32, 42) est disposée sur une seconde zone d'extrémité (36, 46) opposée à la première zone d'extrémité (34, 44).

5. Dispositif de retenue (10) selon la revendication 1,
**caractérisé en ce que**
des zones d'extrémité mutuellement opposées (34, 36, 44, 46) d'au moins un élément haptique parmi les au moins deux éléments haptiques (30, 40) sont reliées au composant de réception (60) et au moins une zone centrale (38, 44) du au moins un élément haptique, disposée entre les zones d'extrémité (34, 36, 44, 46) respectives, est espacée du composant de réception (60), la au moins une zone de saillie (32, 42) étant disposée sur la au moins une zone centrale (38, 48).

6. Dispositif de retenue (10) selon l'une des revendications 1 à 5,
**caractérisé en ce que**
le au moins un composant de réception (60) est déformable élastiquement sous l'action de la force de pression (F) au moyen des au moins deux éléments haptiques (30, 40).

7. Dispositif de retenue (10) selon l'une des revendications 1 à 6,
**caractérisé en ce que**
le au moins un composant de réception (60) comprend au moins une zone de serrage (66) sur laquelle l'implant optique (12) peut être serré et peut ainsi être fixé de manière détachable.

8. Dispositif de retenue (10) selon l'une des revendications précédentes,
**caractérisé en ce que**
le dispositif de retenue (10) est formé d'un seul tenant.

9. Dispositif optique (80) comportant un dispositif de retenue (10) selon l'une des revendications 1 à 8 et un implant optique (12) maintenu sur le dispositif de retenue (10).

10. Dispositif optique (80) selon la revendication 9,
**caractérisé en ce que**
le dispositif optique (80) est formé d'un seul tenant.

11. Dispositif optique (80) selon la revendication 9,
**caractérisé en ce que**
l'implant optique (12) est fixé au dispositif de retenue (10) de manière réversiblement détachable.
